# EUROPEAN PATENT APPLICATION

(11) **EP 4 582 066 A1**
(43) Date of publication of application: **09.07.2025**
(21) Application number: 22957403.3
(22) Date of filing: 31.08.2022
(51) Int. Cl.: A61F 2/80

(54) **SOFT INSERT MANUFACTURING METHOD, DEVICE, AND SYSTEM**

(71) Applicant: INSTALIMB, INC., Tokyo 1300003 (JP)
(72) Inventor: IWANE, Tomoya, Tokyo 130-0003 (JP); KON, Shinichiro, Tokyo 130-0003 (JP); TOKUSHIMA, Yutaka, Tokyo 130-0003 (JP)
(74) Representative: Kilian Kilian & Partner mbB
(86) International application number: PCT/JP2022/032799
(87) International publication number: WO 2024/047805

(57) **Abstract**

A method of manufacturing a soft insert is provided, including: a socket outputting step of outputting a prosthesis socket with a three-dimensional printer based on three-dimensional prosthesis socket shape information; and a suction step of performing suction of a closed space containing the output prosthesis socket and a soft material placed along an inner surface of the prosthesis socket to shape the soft material into a soft insert.

## Description

### Technical Field

This invention relates to a method of manufacturing a soft insert that fits a prosthesis or a prosthesis socket and the like.

### Background Art

A prosthesis user can use a soft insert for purposes such as protecting a stump or improving the fit with the stump when attaching a prosthesis socket to the stump.

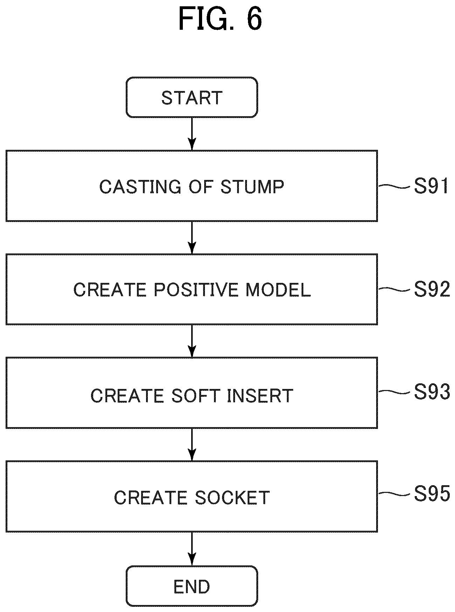

Figure 6 is a flowchart generally illustrating a conventional method of manufacturing a soft insert. As is apparent from the figure, a prosthesis manufacturer first performs casting of the stump of a limb of a person who will wear a prosthesis by wrapping gypsum bandage or the like around the stump (S91).

Following the casting, the prosthesis manufacturer creates a positive model of the stump with gypsum (S92). In doing so, predetermined shape modification may be made manually.

After creating the positive model, the prosthesis manufacturer performs a creation process of a soft insert (S93). More specifically, the prosthesis manufacturer cuts soft material as the material for a soft insert, shapes it into a tubular form and then heats it in an oven. The prosthesis manufacturer then places the heated, now deformable soft material onto the positive model, closes the cut end with soft material and appropriately shapes it such as by cutting or shaving. Through the series of steps, a soft insert made of soft material is formed on the surface of the positive model.

After creation of the soft insert, the prosthesis manufacturer makes the prosthesis socket further outside the soft insert (S95). More specifically, the prosthesis manufacturer lays hard material such as FRP on top of the soft insert, wraps the whole with a plastic bag and the like and connects it to a suction tool to perform a vacuum suction process (a lamination method). Through the series of steps (S91 to S95), a soft insert that fits a prosthesis socket can be manufactured.

More recently, there is also a practice to three-dimensionally scan a stump to acquire its shape, generate a prosthesis shape or prosthesis socket shape from the acquired shape of the stump, and output a prosthesis or a prosthesis socket such as with a three-dimensional printer (Patent Literature 1, for instance).

### Citation List

### Patent Literature

Patent Literature 1
Japanese Patent No. 6948726

### Summary of Invention

### Technical Problem

In cases where formation of a prosthesis socket is directly generated from the result of three-dimensional scanning of the stump and it is output via a three-dimensional printer, such as in Patent Literature 1, no positive model of the stump is created in the manufacture process. In this case, a manufacturing method of soft inserts that is predicated on the presence of a positive model, such as shown in Figure 6, cannot be applied in the manufacture of a soft insert.

The present invention has been made in view of the technical background outlined above and an object thereof is to manufacture a soft insert that fits a prosthesis socket even when the prosthesis socket is manufactured without the need to create a positive model of a stump.

### Solution to Problem

The technical problem described above can be solved by a method, an apparatus, or a system for manufacturing a soft insert having the configurations below.

A method of manufacturing a soft insert according to the present invention includes: a socket outputting step of outputting a prosthesis socket with a three-dimensional printer based on three-dimensional prosthesis socket shape information; and a suction step of performing suction of a closed space containing the output prosthesis socket and a soft material placed along an inner surface of the prosthesis socket to shape the soft material into a soft insert.

Such an arrangement allows a soft insert that fits a prosthesis socket to be manufactured even when the prosthesis socket is manufactured without the need to create a positive model of the stump and the like due to use of a three-dimensional printer. Moreover, since the soft material is shaped by suction, a soft insert with an even thickness can be provided. Here, the inner surface of the prosthesis socket refers to a surface among the surfaces of the prosthesis socket that is on the side of receiving the stump. If the soft material is thermoplastic, it may be placed onto the inner surface of the prosthesis socket after being heated enough to be deformable.

The method may further include a processing shape information generating step of generating work shape information based on the three-dimensional prosthesis socket shape information, the processing shape information being information for processing a shape of the soft material. In the suction step, the soft material shaped based on the processing shape information may be placed so as to fit along the inner surface of the prosthesis socket.

Such an arrangement allows soft material to be processed beforehand into a shape that is easy to fit the prosthesis socket, thus leading to improved work efficiency.

The processing shape may be a trapezoid that is determined based on circumference value information for the prosthesis socket included in the three-dimensional prosthesis socket shape information.

Such an arrangement allows soft material to be processed beforehand so as to be conformable to the shape of the prosthesis socket with a varying diameter, thus leading to improved work efficiency.

The closed space may be suctioned with each of an inner surface of the soft material and a portion or all of an outer surface of the prosthesis socket being covered with predetermined fabric.

Such an arrangement can prevent a failure in suction as a result of breakage of the bag because fabric is placed between the bag and the soft material and between the bag and the prosthesis socket.

The fabric may be stockinet.

Such an arrangement allows an object to be suctioned or the bag to be protected by stockinet made of nylon fiber.

The closed space may be formed using a predetermined bag.

Such an arrangement allows a suction process to be performed with a simple arrangement and using a bag.

The prosthesis socket may further include a through-hole that is formed from a distal end of the prosthesis socket on inner surface side to a distal end face of the prosthesis socket. In the suction step, the closed space may be formed using a predetermined bag, and the closed space may be formed by fixing one end of the bag at or near a distal end of the prosthesis socket through the through-hole from the inner surface side of the prosthesis socket and folding back the bag at a proximal end of the prosthesis socket.

Such an arrangement allows the soft material placed on the inner surface side of the prosthesis socket to be shaped to fit along the inner surface shape by suction.

The one end of the bag may be fixed at or near the distal end of the prosthesis socket by a wire and the wire may be fixed at or near the distal end of the prosthesis socket through the through-hole.

Such an arrangement can achieve shaping along the inner surface of the prosthesis socket with a simple arrangement.

The method may further include a scanning step of acquiring stump shape information with a three-dimensional scanner, the stump shape information being information on a shape of a stump; and a socket shape generating step of generating three-dimensional prosthesis socket shape information based on the stump shape information, the three-dimensional prosthesis socket shape information being three-dimensional shape information for a prosthesis socket that fits the stump. In the socket outputting step, the prosthesis socket may be output based on the three-dimensional prosthesis socket shape information.

According to such an arrangement, a soft insert that fits a prosthesis socket can be manufactured even when the prosthesis socket is manufactured without the need to create a positive model of the stump, such as in the case of acquiring where stump shape information with a three-dimensional scanner, generating three-dimensional prosthesis socket shape information based on it, and performing three-dimensional printing.

The three-dimensional prosthesis socket shape information may be generated by means of a trained model.

Such an arrangement enables the shape of a prosthesis socket that fits the stump to be quickly generated using a machine learning technique.

The soft material may be a thermoplastic material.

Such an arrangement enables shaping that facilitates fitting of the soft material to the inner circumferential surface of the socket by heating.

The thermoplastic material may be polyolefin foam.

Such an arrangement enables shaping that facilitates fitting of the soft material to the inner circumferential surface of the socket.

According to another aspect, the present invention provides an apparatus for manufacturing a soft insert, the apparatus including: a socket output unit that outputs a prosthesis socket with a three-dimensional printer based on three-dimensional prosthesis socket shape information; and a suction unit that performs suction of a closed space containing the output prosthesis socket and a soft material placed along an inner surface of the prosthesis socket to shape the soft material into a soft insert.

According to a further aspect, the present invention provides a system for manufacturing a soft insert, the system including: a socket output unit that outputs a prosthesis socket with a three-dimensional printer based on three-dimensional prosthesis socket shape information; and a suction unit that performs suction of a closed space containing the output prosthesis socket and a soft material placed along an inner surface of the prosthesis socket to shape the soft material into a soft insert.

### Advantageous Effect of Invention

According to the present invention, a soft insert that fits a prosthesis socket can be manufactured even when the prosthesis socket is manufactured without the need to create a positive model of the stump.

### Brief Description of Drawings

[Figure 1] Figure 1 is a general flowchart illustrating a manufacturing process of a prosthetic leg socket and a soft insert.
[Figure 2] Figure 2 is a detailed flowchart illustrating the manufacturing process of a soft insert.
[Figure 3] Figure 3 illustrates soft insert shape.
[Figure 4] Figure 4 illustrates the way of covering the prosthetic leg socket with stockinet.
[Figure 5] Figure 5 illustrates a cross section of the prosthetic leg socket and other components with a bag placed thereon.
[Figure 6] Figure 6 is a flowchart generally illustrating a conventional method of manufacturing a soft insert.

### Description of Embodiments

Preferred embodiments of the present invention are now described in detail with reference to the attached drawings.

### (1. First embodiment)

As a first embodiment, an example of the present invention as applied to a soft insert that fits a transtibial prosthesis socket (hereinafter, a prosthetic leg socket) is described.

While the present embodiment describes a transtibial prosthesis socket as a prosthesis socket, the present invention is not limited to such an arrangement. Accordingly, the techniques according to the present embodiment may be applied to other prosthetic legs such as a transfemoral prosthetic leg and a hip disarticulation prosthesis, for example. It may also be applied to sockets for other stumps of a body, such as an arm and a finger.

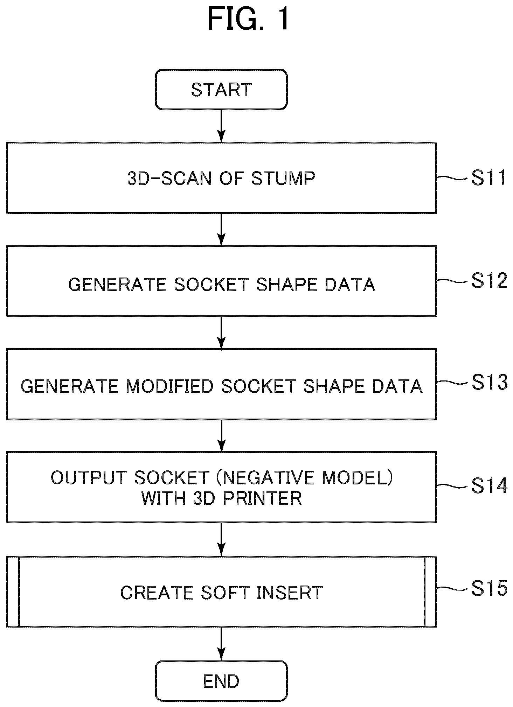

Figure 1 is a general flowchart illustrating the manufacturing process of a prosthetic leg socket 1 and a soft insert 2 according to the present embodiment. As is apparent from the figure, a prosthetic leg manufacturer first scans a stump of a person who wants to use the prosthetic leg socket 1 with a three-dimensional scanner (3D scanner) to acquire shape data for the stump (S11). The shape data for the stump acquired by the three-dimensional scanner goes through predetermined post-processing and the like to be stored in a connected information processing device, not shown.

The information processing device in the present embodiment is a PC (personal computer) that includes a control unit such as a CPU/GPU for processing execution of programs, a storage unit for storing programs and data, an I/O unit and a communication unit to interface with other devices. The present invention is not limited to such a configuration but may use other kind of information processing device such as a tablet terminal.

After the three-dimensional scanning of the stump, the prosthetic leg manufacturer operates the information processing device to generate prosthetic leg socket shape data based on the stump shape data (S12).

In the present embodiment, for generation of prosthetic leg socket shape data, a trained model that has learned the relationship between stump shape data and prosthetic leg socket shape data that suits the stump of interest is used. That is, the prosthetic leg manufacturer generates (or infers) prosthetic leg socket shape data that suits the stump shape data of interest by performing an operation to enter the stump shape data to the trained model.

For the trained model, any of various known techniques can be employed. For example, a trained model can be obtained by supervised learning of a neural network.

Such an arrangement enables the shape of a prosthesis socket that fits the stump to be quickly generated using a machine learning technique having generalization effect.

In the present embodiment, the prosthetic leg socket 1 is a socket to receive the lower thigh and has a cylindrical protrusion with a through-hole portion provided in the center at its distal end. This cylindrical protrusion can be inserted into an adapter and the like and fixed, thus manufacturing a prosthetic leg.

The present invention is not limited to such an arrangement. Thus, the present invention may be applied to a coupling portion of a prosthesis resembling the entire missing body part with the stump, for example. The prosthetic leg socket 1 may also be a test socket, for example, not a final product for use. Furthermore, the cylindrical protrusion may not be provided at the distal end of the prosthetic leg socket 1. In short, the tip of the prosthetic leg socket 1 may be simply a flat surface or a curved surface, as long as the distal end of the prosthetic leg socket 1 or an adjacent portion can be coupled to an adapter and the like. In addition, different shapes may be used, such as a cylindrical protrusion is provided at the distal end of a test socket and the distal end of the final socket is a flat surface.

The way of generating prosthetic leg socket shape data is not limited to such an arrangement. Hence, the prosthetic leg manufacturer may generate it by three-dimensional CAD and the like while checking the conformance with the stump shape data, for example. Moreover, prosthetic leg socket shape data may be output with a rule base based on the stump shape data without using a trained model.

After generating the prosthetic leg socket shape data, the prosthetic leg manufacturer performs processing to make predetermined modification to the generated prosthetic leg socket shape data to generate modified socket shape data (S13). The modification in the present embodiment is processing to increase the inner diameter of the prosthetic leg socket shape by a predefined amount, that is, processing to generate shape data for a somewhat larger prosthetic leg socket. The modification may be made by the manufacturer by three-dimensional CAD and the like or the information processing device may automatically make a shape modification by a predetermined amount.

This arrangement allows for generation of a somewhat larger prosthetic leg socket shape, so the right fit can be provided when the soft insert is attached to the inside of the prosthetic leg socket as discussed later.

For generation of shape data for a somewhat larger prosthetic leg socket, a trained model may learn the relationship between the stump shape data and shape data for a prosthetic leg socket with a slightly larger inner dimeter than the shape that suits the shape data, and shape data for a somewhat larger prosthetic leg socket may be directly generated from the stump shape data.

The amount of modification to the prosthetic leg socket shape may be determined on the basis of various other parameters separately acquired. The parameters can include stump shape data, the BMI value of the prosthesis user, and deep part information (such as percentage of body fat), for example.

After generating the prosthetic leg socket shape data, the prosthetic leg manufacturer performs processing to output the prosthetic leg socket 1 based on the prosthetic leg socket shape data using a three-dimensional printer connected to the information processing device (S14). The prosthetic leg socket 1 is output using hard material such as FRP (fiber reinforced plastics).

The prosthetic leg manufacturer then creates the soft insert 2 based on the prosthetic leg socket 1 and the prosthetic leg socket shape data (S15).

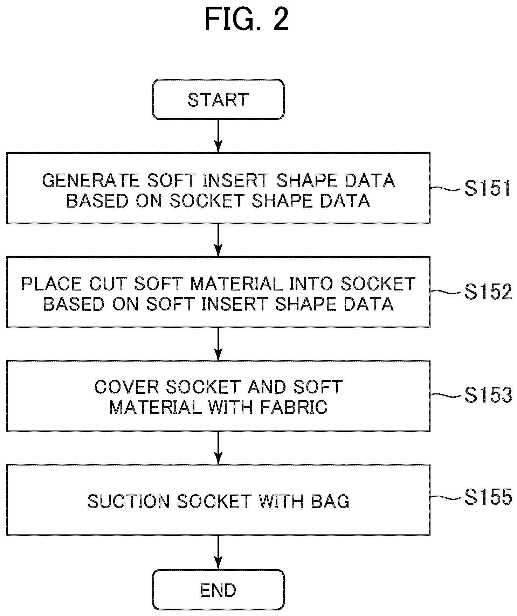

Figure 2 is a detailed flowchart illustrating the manufacturing process (S15) of the soft insert 2 according to the present embodiment. For manufacture of the soft insert 2, the prosthetic leg manufacturer first generates soft insert shape data based on the prosthetic leg socket shape data using the information processing device (S151). In the present embodiment, soft insert shape data is two-dimensional shape data for cutting a plate of soft material. The soft material is material with thermoplasticity, such as polyolefin foam (e.g., PE Lite), for example. The material with thermoplasticity may also be polyurethane foam and the like. Such soft materials may also be called cushion material.

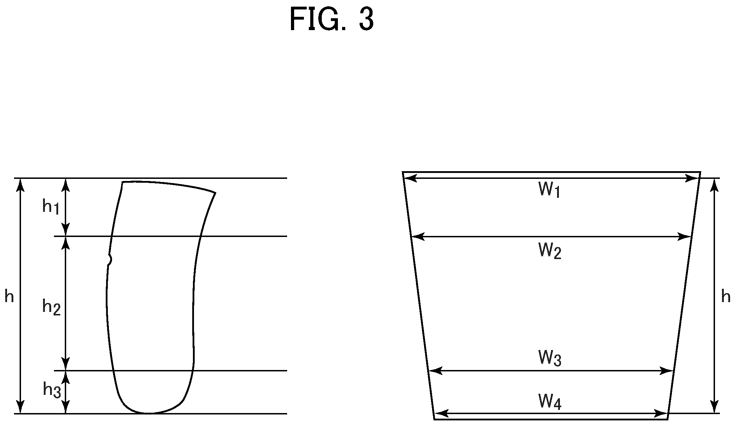

Figure 3 illustrates soft insert shape. On the left-hand side of the figure, a prosthetic leg socket shape is depicted, and on the right-hand side of the figure, a two-dimensional shape for cutting out a plate of polyolefin foam corresponding to the prosthetic leg socket shape is depicted.

As is apparent from the figure, the two-dimensional shape in the present embodiment is a trapezoid shape with the lower base (the width of the distal end) being shorter than the upper base (the width of the proximal end). The height h of the trapezoid shape is calculated based on the longitudinal length of the prosthetic leg socket.

The width of the trapezoid is calculated based on the circumference value (the outer diameter value or inner diameter value) of the prosthetic leg socket at a certain point. That is, a width W1 of the trapezoid is calculated based on the inner diameter or the outer diameter of the prosthetic leg socket at the proximal end. A width W2 of the trapezoid is calculated based on the outer diameter or the inner diameter of the prosthetic leg socket at a point at a distance of h1 from the proximal end of the prosthetic leg socket. A width W3 of the trapezoid is calculated based on the outer diameter or the inner diameter of the prosthetic leg socket at a point at a distance of h1 + h2 from the proximal end of the prosthetic leg socket. A width W4 of the trapezoid is defined as the width at a distance of h from the proximal end on an extension of the trapezoid that is defined by the width W2, which is at a distance of h1 from the proximal end, and the width W3, which is at a distance of h1 + h2 from the proximal end.

Such an arrangement allows soft material to be processed beforehand into a shape that is easy to fit the prosthesis socket 1, thus leading to improved work efficiency. The work efficiency is improved particularly in the case of cutting into a trapezoid shape because soft material can be cut out beforehand so as to be conformable to the shape of the prosthesis socket, which is tapered toward the distal end.

After the generation process of soft insert shape data, the prosthetic leg manufacturer cuts out the soft material based on the soft insert shape data and places it so as to fit along the inner surface side of the prosthetic leg socket (S152).

More specifically, the prosthetic leg manufacturer uses a predetermined processing tool or a machine tool to cut a plate of soft material into a trapezoid shape based on the soft insert shape data. The cut soft material in the trapezoid shape is rolled and bonded at the ends into a tubular shape. The prosthetic leg manufacturer places the now tubular soft material 2 into an oven and the like and heats it into a deformable state, and then places the soft material 2 so as to fit along the inner surface (curved surface) of the prosthetic leg socket 1 output by a three-dimensional printer. The inner surface refers to a surface among the surfaces of the prosthetic leg socket 1 that makes contact with or faces the stump.

After that, the prosthetic leg manufacturer covers the surfaces of the prosthetic leg socket 1 and the soft material 2 with fabric (S153). More specifically, in the present embodiment, the outer surface of the prosthetic leg socket 1 and the inner surface of the soft material 2 are covered. In the present embodiment, the fabric is stockinet 5 as an example.

Such an arrangement allows an object to be suctioned or a bag 3, discussed later, to be protected by stockinet 5 made of nylon fiber.

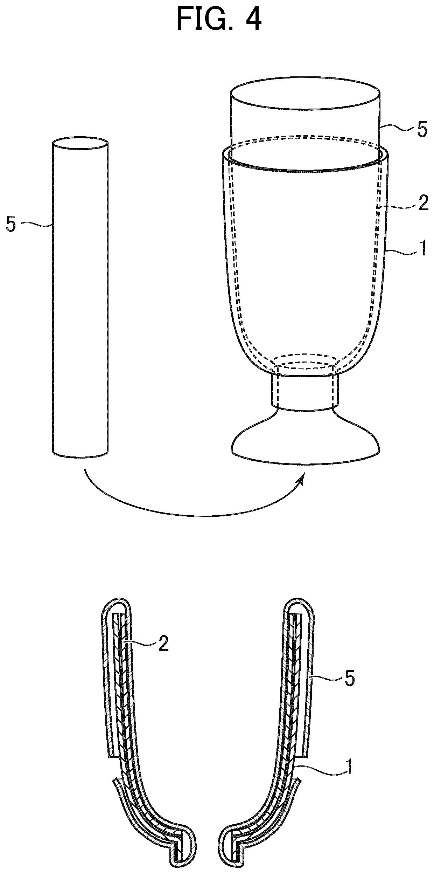

Figure 4 illustrates the way of covering the prosthetic leg socket 1 with the stockinet 5. In the upper portion of the figure, how a tubular stockinet 5 is inserted through a through-hole portion formed in the bottom of the prosthetic leg socket 1. In the lower portion of the figure, a cross-sectional view of the prosthetic leg socket 1 and other components with the stockinet 5 folded back is depicted. Note that the figure is a conceptual view for illustration and is not an exact depiction of the shape.

For covering, the tubular stockinet 5 is first inserted into the through-hole portion formed in the bottom of the prosthetic leg socket 1, as shown in the upper portion of the figure. In this condition, the proximal and distal ends of the inserted stockinet 5 are folded back to cover the inner and outer surfaces of the prosthetic leg socket 1, as shown in the lower portion of the figure.

While in the present embodiment, both the proximal and distal ends of the stockinet 5 are folded back, the present invention is not limited to such an arrangement. Only one of the proximal and distal ends may be folded back to cover the surfaces, as long as the outer surface of the prosthetic leg socket 1 and the inner surface of the prosthetic leg socket 1, on which the soft insert 2 is placed, can be protected.

After covering the prosthetic leg socket 1 with the stockinet 5, the prosthetic leg manufacturer performs processing to suck the content using a bag 3 with flexibility (S155). The present embodiment uses an easily available bag made of polyethylene (a polyethylene bag) as an example of the bag 3. The bag 3 is not limited to one made of polyethylene. A bag 3 made of other material such as polypropylene and vinyl chloride resin (a plastic bag) may be used as long as it has flexibility and withstand suction.

More specifically, the prosthetic leg manufacturer first brings a wire 4 attached to a portion of the bag 3 close to the prosthetic leg socket 1 from the side of the inner surface of the prosthetic leg socket 1 and inserts it midway down the through-hole portion at the distal end of the prosthetic leg socket 1. He then ties and fixes the wire 4 to the cylindrical portion provided at the distal end of the prosthetic leg socket 1. This results in an end of the bag 3 being fixed near the through-hole portion provided at the distal end of the prosthetic leg socket 1, facilitating the bag 3 appropriately fitting along the inner surface of the prosthetic leg socket 1 upon suction, as discussed later.

While in the present embodiment, the point of fixation of the wire 4 to the prosthetic leg socket 1 is the cylindrical portion, the present invention is not limited to such an arrangement. The wire 4 may be fixed at any point as long as it is at or near the distal end of the prosthetic leg socket 1.

The prosthetic leg manufacturer then folds back the bag 3 at the proximal end of the prosthetic leg socket 1, and connects the open end of the bag 3 to a suction tool to form a suction path. That is, a closed space is thereby formed within the bag 3 and a suction-enabled state is created.

Finally, the bag 3 is subjected to a suction process until the soft insert 2 is sufficiently shaped. The suction process may be a suction process using a vacuum pump, for example.

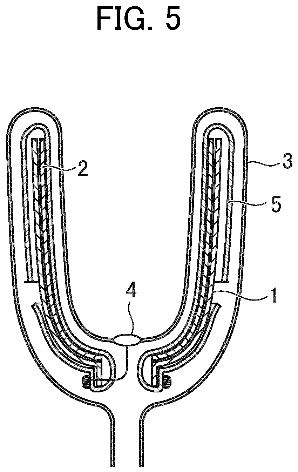

Figure 5 illustrates a cross section of the prosthetic leg socket 1 and other components with the bag 3 placed thereon. Note that the figure is a conceptual view for illustration and is not an exact depiction of the shape.

As is apparent from the figure, the wire 4 is attached near the through-hole portion at the distal end of the prosthetic leg socket 1 on the inner surface side, and one end of the wire 4 is passes through a through-hole formed in the side surface of the cylindrical protrusion provided at the distal end of the prosthetic leg socket 1 and then wound and fixed around the cylindrical protrusion.

The bag 3 is folded back at the proximal end of the prosthetic leg socket 1. The open end of the bag 3 is connected to a suction tool (not shown) near the cylindrical protrusion at the distal end of the prosthetic leg socket 1.

Such an arrangement allows the inner and outer surfaces of the prosthetic leg socket 1, or the contents of the bag 3, to be evenly pressurized by suction. Consequently, a soft insert 2 having a shape along the inner surface profile of the prosthetic leg socket 1 and being even in thickness can be provided.

Such an arrangement also can prevent direct transmission of heat and the like of the soft material 2 to the bag 3 and prevent breakage of the bag 3 because the fabric 5 (the stockinet 5) is placed between the bag 3 and the inner surface of the soft material 2. Additionally, since the fabric 5 (the stockinet 5) is placed between the bag 3 and the outer surface of the prosthesis socket 1, layering marks on the prosthetic leg socket 1 or breakage of the bag 3 due to a burr caused by the use of a three-dimensional printer can be prevented. That is to say, a failure in suction as a result of breakage of the bag 3 and the like can be prevented.

Furthermore, such an arrangement can easily achieve shaping along the inner surface of the prosthesis socket 1 because the wire 4 can be used to place the bag 3 such that it can be more easily fitted to the inner surface of the socket 1.

Thus, from the foregoing, a soft insert that fits a prosthesis socket can be manufactured even when the prosthesis socket is manufactured without the need to create a positive model of the stump due to use of a three-dimensional printer.

### (2. Variation)

The present invention can be practiced with different modifications.

Although in the embodiment above the prosthesis manufacturer was described as operating an information processing device or the like to perform each step, all the steps may be automatically performed through coordination of a three-dimensional scanner, an information processing device, a three-dimensional printer, a machine tool and the like, without being subjected to operations by the prosthetic leg manufacturer. In this case, these arrangements are perceived as an apparatus or a system for manufacturing a prosthesis and a soft insert that fits the prosthesis.

While the embodiments of the present invention have been described, the above embodiments only show some of possible applications of the present invention and is not intended to limit the technical scope of the present invention to the specific configurations of the embodiments. In addition, the embodiments described above may be combined unless they are inconsistent.

### Industrial Applicability

The present invention is applicable in industries that manufacture prostheses, soft inserts and the like.

### Reference Signs List

- 1: prosthetic leg socket (prosthesis socket)
- 2: soft material (soft insert)
- 3: bag
- 4: wire
- 5: stockinet (fabric)

## Claims

1. A method of manufacturing a soft insert, the method comprising:
a socket outputting step of outputting a prosthesis socket with a three-dimensional printer based on three-dimensional prosthesis socket shape information; and
a suction step of performing suction of a closed space containing the output prosthesis socket and a soft material placed along an inner surface of the prosthesis socket to shape the soft material into a soft insert.

2. The manufacturing method according to claim 1, further comprising:
a processing shape information generating step of generating processing shape information based on the three-dimensional prosthesis socket shape information, the processing shape information being information for processing a shape of the soft material, wherein
in the suction step, the soft material shaped based on the processing shape information is placed so as to fit along the inner surface of the prosthesis socket.

3. The manufacturing method according to claim 2, wherein the processing shape is a trapezoid that is determined based on circumference value information for the prosthesis socket included in the three-dimensional prosthesis socket shape information.

4. The manufacturing method according to claim 1, wherein the closed space is suctioned with each of an inner surface of the soft material and a portion or all of an outer surface of the prosthesis socket being covered with predetermined fabric.

5. The manufacturing method according to claim 4, wherein the fabric is stockinet.

6. The manufacturing method according to claim 4, wherein the closed space is formed using a predetermined bag.

7. The manufacturing method according to claim 1, wherein
the prosthesis socket further comprises a through-hole that is formed from a distal end of the prosthesis socket on inner surface side to a distal end face of the prosthesis socket, and
in the suction step, the closed space is formed using a predetermined bag, and the closed space is formed by fixing one end of the bag at or near a distal end of the prosthesis socket through the through-hole from the inner surface side of the prosthesis socket and folding back the bag at a proximal end of the prosthesis socket.

8. The manufacturing method according to claim 7, wherein the one end of the bag is fixed at or near the distal end of the prosthesis socket by a wire, and the wire is fixed at or near the distal end of the prosthesis socket through the through-hole.

9. The manufacturing method according to claim 1, further comprising:
a scanning step of acquiring stump shape information with a three-dimensional scanner, the stump shape information being information on a shape of a stump; and
a socket shape generating step of generating three-dimensional prosthesis socket shape information based on the stump shape information, the three-dimensional prosthesis socket shape information being three-dimensional shape information for a prosthesis socket that fits the stump, wherein
in the socket outputting step, the prosthesis socket is output based on the three-dimensional prosthesis socket shape information.

10. The manufacturing method according to claim 9, wherein the three-dimensional prosthesis socket shape information is generated by means of a trained model.

11. The manufacturing method according to claim 1, wherein the soft material is a thermoplastic material.

12. The manufacturing method according to claim 11, wherein the thermoplastic material is polyolefin foam.

13. An apparatus for manufacturing a soft insert, the apparatus comprising:
a socket output unit that outputs a prosthesis socket with a three-dimensional printer based on three-dimensional prosthesis socket shape information; and
a suction unit that performs suction of a closed space containing the output prosthesis socket and a soft material placed along an inner surface of the prosthesis socket to shape the soft material into a soft insert.

14. A system for manufacturing a soft insert, the system comprising:
a socket output unit that outputs a prosthesis socket with a three-dimensional printer based on three-dimensional prosthesis socket shape information; and
a suction unit that performs suction of a closed space containing the output prosthesis socket and a soft material placed along an inner surface of the prosthesis socket to shape the soft material into a soft insert.
